# FASCICULE DE BREVET EUROPEEN

(11) **EP 0 803 503 B1**
(45) Date de publication et mention de la délivrance du brevet: **03.11.1999**
(21) Numéro de dépôt: 97400912.8
(22) Date de dépôt: 23.04.1997
(51) Int. Cl.: C07D 311/30, A61K 31/35

(54) **Acides et esters de la diosmétine et compositions pharmaceutiques les contenant**
Diosmetin-Säuren und Ester und diese enthaltende pharmazeutische Zusammensetzungen
Acids and esters of diosmetin and pharmaceutical compositions containing them

(30) Priorité: 25.04.1996 FR 9605247
(43) Date de publication de la demande: 29.10.1997
(73) Titulaire: ADIR ET COMPAGNIE, 92415 Courbevoie Cédex (FR)
(72) Inventeur: Wierzbicki, Michel, 78620 L'Etang La Ville (FR); Boussard, Marie-Françoise, 78124 Mareil sur Mauldre (FR); Verbeuren, Tony, 78540 Vernouillet (FR); Vallez, Marie-Odile, 77420 Champs sur Marne (FR)
(74) Mandataire: Reverbori, Marcelle

(56) Documents cités:
- EP-A- 0 319 412
- FR-A- 2 081 569
- GB-A- 929 522
- CHEMICAL ABSTRACTS, vol. 81, no. 19, 11 Novembre 1974 Columbus, Ohio, US; abstract no. 120382v, XP002021529 & HUA HSUEH, no. 4, 1973, pages 135-136, C.-F. YAN ET AL.:

## Description

La présente invention a pour objet de nouveaux acides et esters de la diosmétine, et les compositions pharmaceutiques les contenant.

Elle concerne plus particulièrement les composés de formule (I): 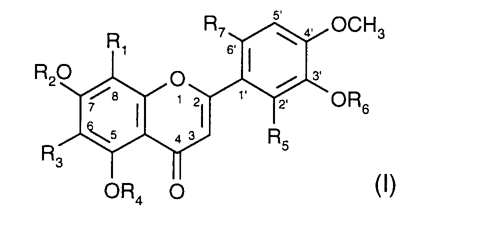 dans laquelle :
- R₂, R₄ et R₆, identiques ou différents, représentent chacun un atome d'hydrogène, un radical alkyle de 1 à 5 atomes de carbone en chaine droite ou ramifiée, ou un radical de formule : 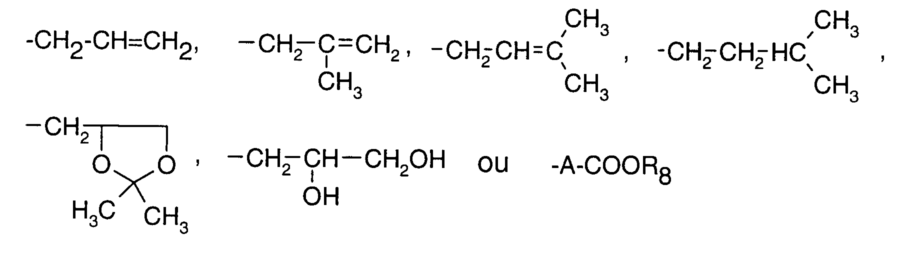 dans lesquelles :
   - A représente une chaîne hydrocarbonée droite de contenant de 1 à 3 atomes de carbone éventuellement substituée par un radical méthyle, ou par un radical hydroxy ;
   - R₈ représente un atome d'hydrogène ou un radical alkyle de 1 à 5 atomes de carbone et
- R₃ et R₅, identiques ou différents représentent chacun un atome d'hydrogène ou un radical de formule : 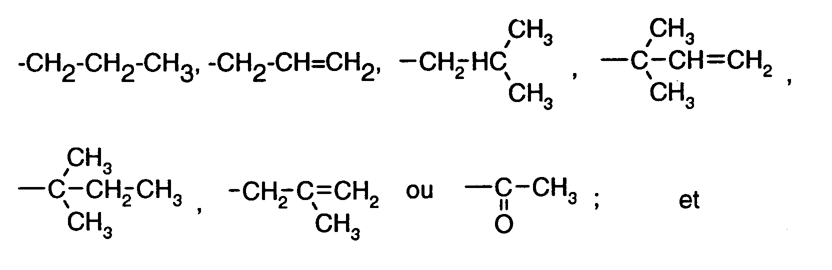
- R₁ et R₇, identiques ou différents, représentent chacun un atome d'hydrogène ou un radical de formule : 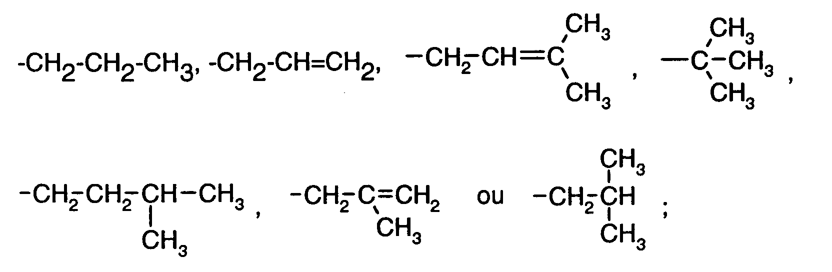
- à condition toutefois que :
- l'un au moins des substituants R₂, R₄ et R₆ représente le groupe -A-COOR₈, et
- lorsque R₂, et/ou R₄, et/ou R₆ représente -A-COOR₈, (A et R₈ ayant les significations précédemment définies), alors un au moins des substituants restants, parmi R₁ à R₇, ne représente pas un atome d'hydrogène;
   ainsi que leurs énantiomères quand ils existent et leurs sels physiologiquement tolérables avec des bases appropriées.

L'état antérieur de la technique est illustré notamment par le brevet EP 0 319 412 qui concerne des dérivés flavonoïdes 2-pipérazinyl 2-oxo éthylène substitués, utilisables pour le traitement des affections vasculaires.

Or la prolifération, sans cesse accrue, de ces affections justifie un accueil favorable à toute invention de médicaments dans ce domaine. C'est dans ce cadre que s'insère la présente invention qui concerne une famille de produits de structures chimiques bien différenciées par rapport à celles des principes actifs antérieurement connus et qui présentent des propriétés pharmacologiques et thérapeutiques particulièrement intéressantes pour le traitement de ces dites affections.

La présence d'une microangiopathie capillaire-veinulaire spécifique a été mise en évidence dans les insuffisances veineuses chroniques. Cette microangiopathie, qui est la conséquence de l'hypertension veineuse, entraîne des troubles de filtration capillaire-veinulaire (hyperméabilité) et donc des micro-oedèmes (Barbier et al., La Presse Médicale, 23, p. 213-224, 1994). L'amélioration des troubles micro circulatoires qui accompagnent l'insuffisance veineuse chronique (oedème, inflammation) doit faire partie du traitement médicamenteux de cette maladie (Chauveau, La Presse Médicale, 23, p. 243-249, 1994).

Or, il a été trouvé, dans les services de la demanderesse, que les composés objet de la présente invention possèdent une activité anti-hyperperméabilité mise en évidence en utilisant des techniques modernes en microscopie afin d'évaluer les réponses de la micro circulation, cf Bjork et al., Progr. Appl. Microcircul., 6, 41-53, (1984). Ainsi, les composés de la présente invention sont utilisables notamment dans le traitement de l'insuffisance veineuse chronique et dans toute autre maladie entraînant une activité d'hyperperméabilité et/ou d'inflammation.

La présente invention a également pour objet les compositions pharmaceutiques contenant comme principe actif un composé de formule I ou un de ses sels physiologiquement tolérable, mélangé ou associé à un ou plusieurs excipients pharmaceutiques appropriés.

Les compositions pharmaceutiques ainsi obtenues se présentent généralement sous forme dosée renfermant de 1 à 500 mg de principe actif. Elles peuvent, par exemple, revêtir la forme de comprimés, dragées, gélules, suppositoires, solutions injectables ou buvables et être administrées par la voie orale, rectale ou parentérale.

La posologie peut varier notablement selon l'âge et le poids du patient, la voie d'administration, la nature de la maladie et les traitements associés et s'échelonne de 1 à 500 mg de principe actif, 1 à 6 fois par jour.

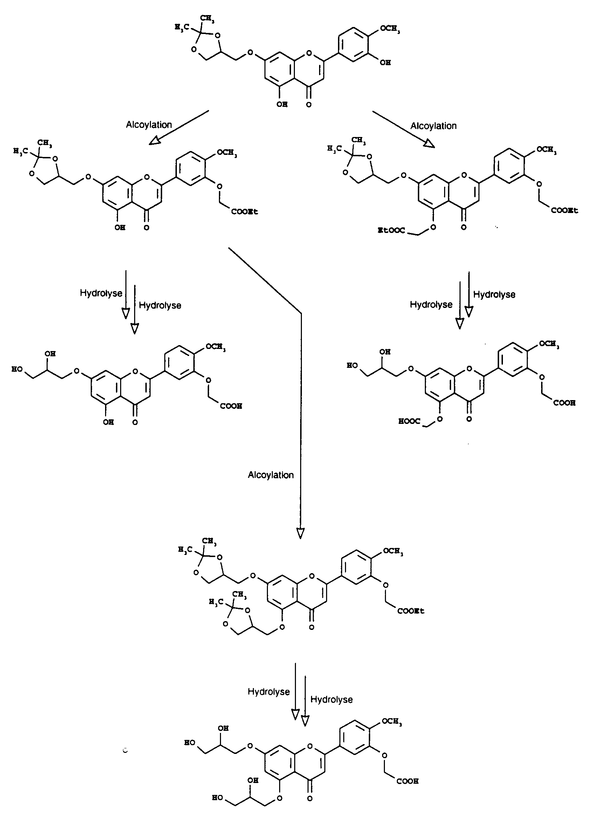

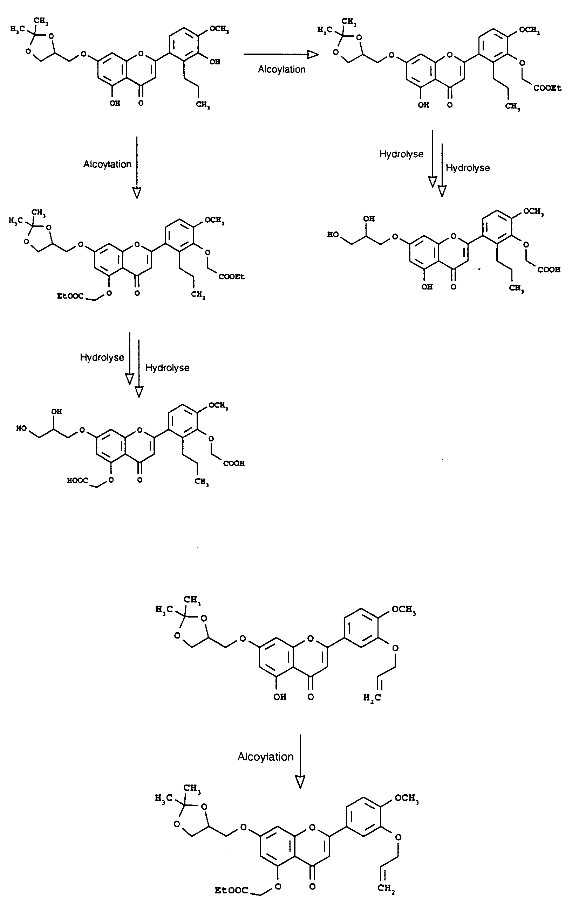

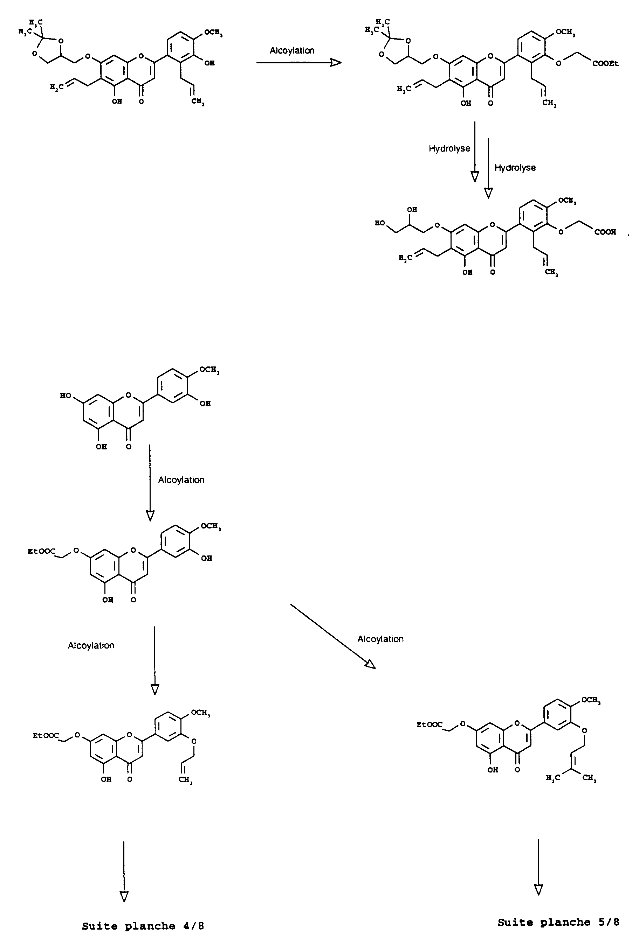

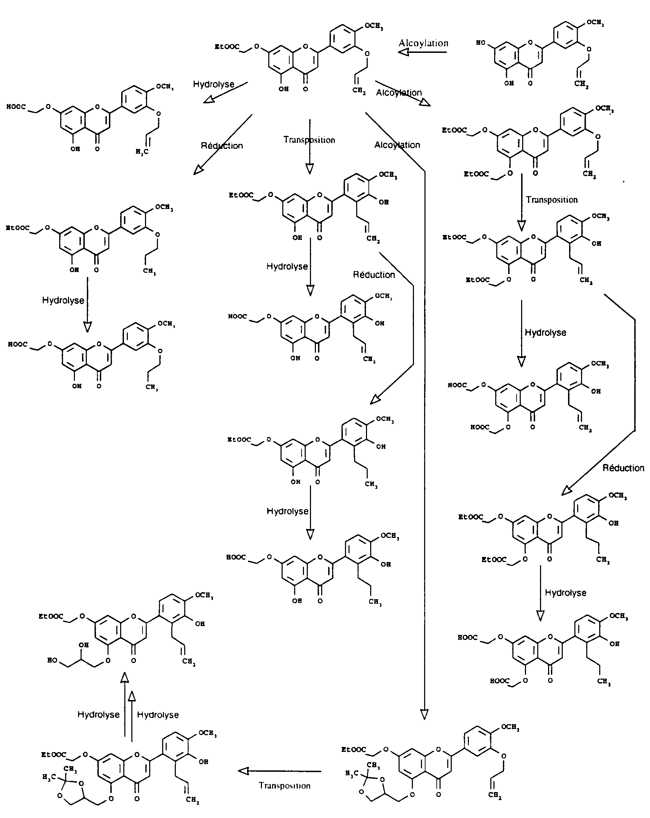

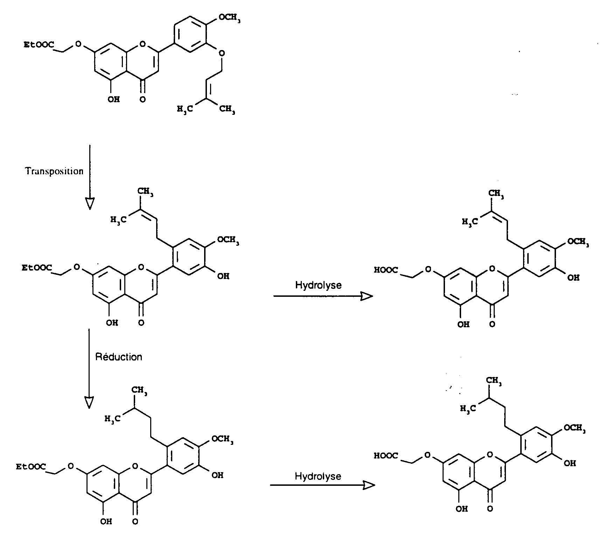

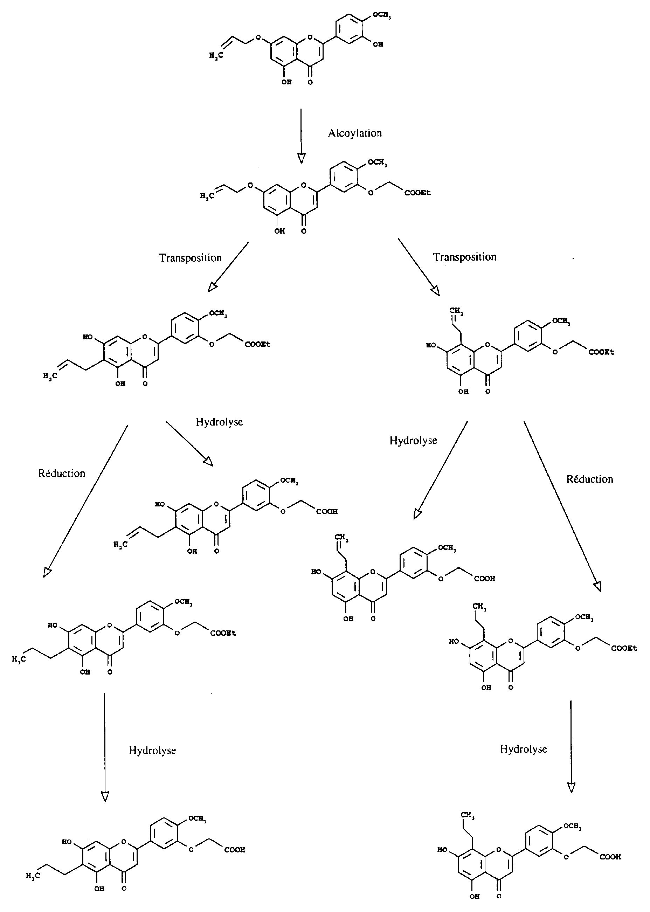

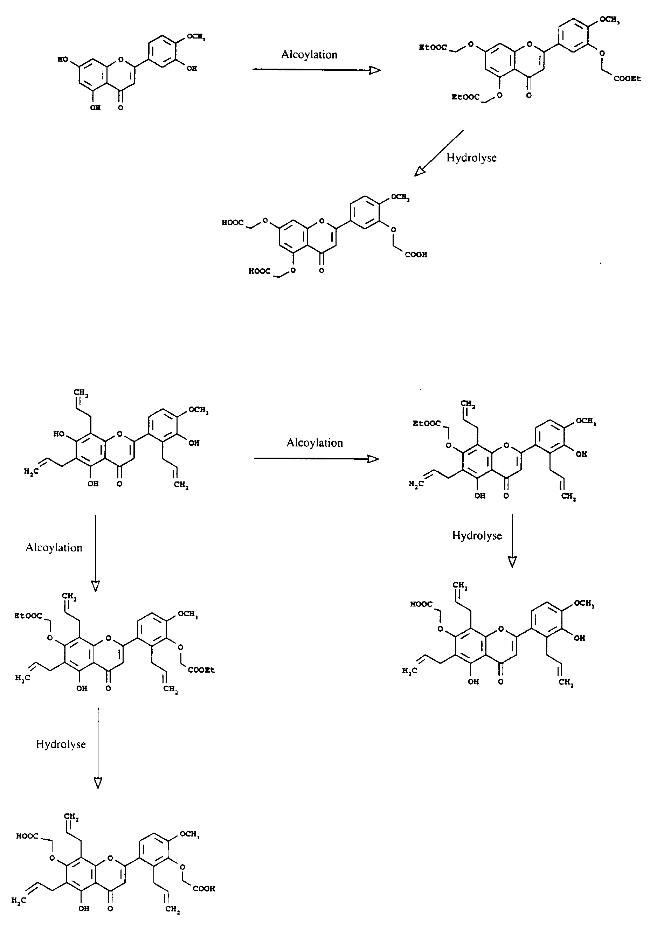

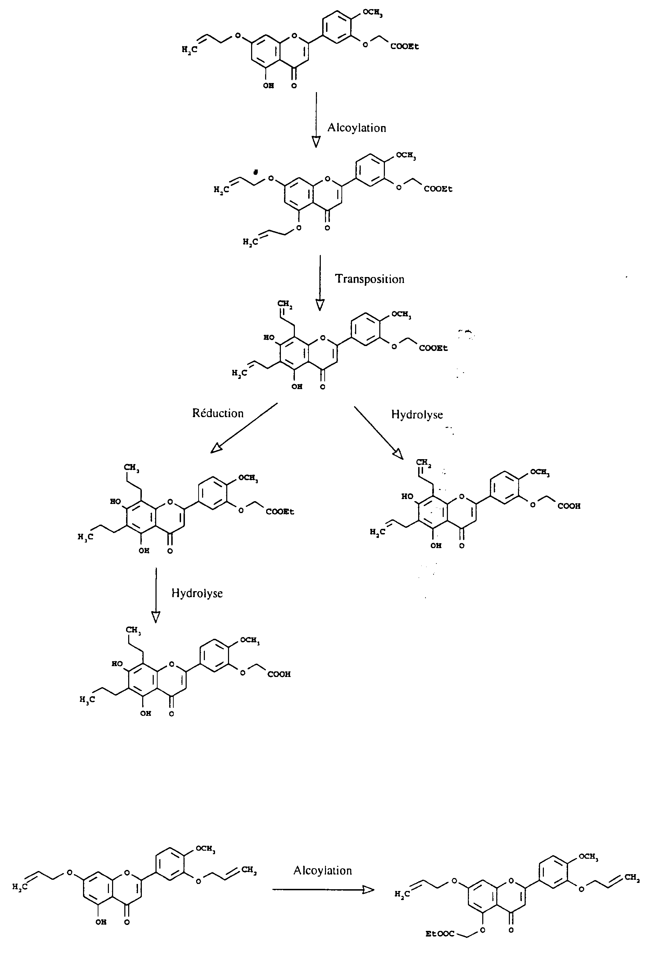

Les composés de formule I sont tous préparés à partir de matières premières connues, en utilisant des méthodes classiques mettant en jeu par exemple, selon les cas, des réactions choisies parmi les réactions d'alcoylation, réduction, transposition, réarrangement, hydrolyse et estérification, comme illustré dans les planches 1 à 8.
- Les réactions d'alcoylation sont réalisées dans l'acétone ou le diméthylformamide, en utilisant comme base un carbonate ou un bicarbonate alcalin (de sodium ou de potassium par exemple), sauf dans le cas où l'alcoylation conceme le groupement OR₄ dans lequel R₄ représente un atome d'hydrogène, où l'on utilise alors le tertiobutylate de potassium ou l'hydrure de sodium dans un mélange de diméthylformamide et d'hexaméthyl phosphortriamide. L'agent alcoylant utilisé est soit un halogénoester, soit le tosylate de glycérol-2,3-acétonide, soit un halogénure d'alkyle ou d'alkényle.
- Les réactions de transposition sont réalisées en chauffant le produit à reflux dans un solvant à haut point d'ébullition comme le trichlorobenzène pour les transpositions en ortho-, ou comme la N,N-diméthylaniline pour les transpositions en para.
- Les réactions de réarrangement sont réalisées en présence d'acide de Lewis comme le chlorure d'aluminium dans un solvant non réactif comme le nitrobenzène ou le chlorobenzène.
- Les réactions de réduction sont réalisées sous pression d'hydrogène en présence de catalyseur tel que le palladium sur charbon.
- Les réactions d'hydrolyse d'esters sont réalisées en saponifiant le composé en présence de soude dans un mélange eau/éthanol suivi d'une acidification par exemple à l'acide chlorhydrique dilué.
- Les réactions d'hydrolyse d'acétonide sont réalisées en chauffant le composé dans un mélange eau/acide acétique.
- Dans le cas où le composé contient à la fois une fonction ester et une fonction acétonide, on peut réaliser les deux hydrolyses successivement en commençant par la fonction ester, mais sans isoler le composé acide-acétonide intermédiaire.
- Les réactions d'estérification sont réalisées en faisant réagir le phénol concerné avec un chlorure d'acide en présence d'une base comme la pyridine ou la triéthylamine.

Les exemples suivants illustrent la présente invention

### Exemple 1:

7-carboxyméthoxy-5-hydroxy-2-(3-hydroxy-4-méthoxy-2-propylphényl)-4H-1-benzopyran-4-one

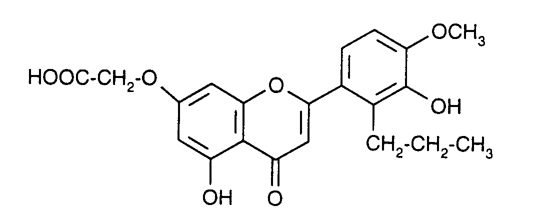

### Stade A : 7-éthoxycarbonylméthoxy-5-hydroxy-2-(3-allyloxy-4-méthoxy phényl)-4H-1-benzopyran-4-one

### α) Première méthode

386 g de 7-éthoxycarbonylméthoxy-5-hydroxy-2-(3-hydroxy-4-méthoxy phényl)-4H-1-benzopyran-4-one (décrit dans EP 0 319 412) sont traités par 165 g de K₂CO₃ dans 2300 ml de diméthylformamide pendant 24 heures.
On additionne ensuite au mélange 157 g de bromure d'allyle et porte le tout à 80°C pendant 24 heures. On laisse revenir à température ambiante, puis on filtre les minéraux formés et élimine le solvant et les parties volatiles par distillation sous pression réduite. L'huile résiduelle obtenue est reprise par l'éther éthylique. On élimine l'insoluble. La phase éthérée est amenée à sec par distillation et le résidu obtenu est recristallisé dans l'isopropanol. On obtient ainsi (avec un rendement de 66%) 281 g du produit titre du stade A avec une pureté >95% (P.F. d'un échantillon analytique : 171°C).

de la même manière, ont été préparés les composés suivants :
a) 7-éthoxycarbonylméthoxy-5-hydroxy-2-[4-méthoxy-3-(3-méthylbut-2-ényloxy) phényl]-4H-1-benzopyran-4-one, P.F. : 154°C, à partir de 7-éthoxycarbonylméthoxy-5-hydroxy-2-(3-hydroxy-4 méthoxy phényl)-4H-1-benzopyran-4-one (cf:EP 0 319 412) et de 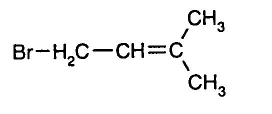
b) 5,7-di-allyloxy-2-(3-éthoxycarbonylméthoxy-4-méthoxy phényl)-4H-1-benzopyran-4-one, P.F. : 105-107°C, à partir de 7-allyloxy-5-hydroxy-2-(3-éthoxycarbonylméthoxy-4-méthoxy phényl)-4H-1-benzopyran-4-one (décrit dans la présente demande) et de Br-CH₂-CH=CH₂ et NaH.
c) 7-(2,2-diméthyl-1,3-dioxol-4-yl méthoxy)-5-hydroxy-2-(3-éthoxycarbonyl méthoxy-4-méthoxy phényl)-4H-1-benzopyran-4-one, P.F. : 104-105°C, à partir de 7-(2,2-diméthyl-1,3-dioxol-4-yl méthoxy)-5-hydroxy-2-(3-hydroxy-4-méthoxy phényl)-4H-1-benzopyran-4-one, (cf la demande de brevet EP 709 383) et de Br-CH₂-COOC₂H₅.
d) 7-(2,2-diméthyl-1,3-dioxol-4-yl méthoxy)-5-éthoxycarbonylméthoxy-2-(3-éthoxycarbonylméthoxy-4-méthoxy phényl)-4H-1 benzopyran-4-one, P.F. : 99°C, à partir de 7-(2,2-diméthyl-1,3-dioxol-4-yl méthoxy)-5-hydroxy-2-(3-hydroxy-4-méthoxy phényl)-4H-1-benzopyran-4-one (cf la demande de brevet EP 709 383) et de Br-CH₂-COOC₂H₅ et NaH.
e) 7-(2,2-diméthyl-1,3-dioxol-4-yl méthoxy)-5-hydroxy-2-(3-éthoxycarbonyl méthoxy-4-méthoxy-2-propyl phényl)-4H-1-benzopyran-4-one P.F. : 76°C, à partir de 7-(2,2-diméthyl-1,3-dioxol-4-yl méthoxy)-5-hydroxy-2-(3-hydroxy-4-méthoxy-2-propyl phényl)-4H-1-benzopyran-4-one (cf la demande de brevet EP 709 383) et de Br-CH₂-COOC₂H₅.
f) 7-(2,2-diméthyl-1,3-dioxol-4-yl méthoxy)-5-éthoxycarbonylméthoxy-2-(3-éthoxycarbonylméthoxy-4-méthoxy-2-propyl phényl)-4H-1-benzopyran-4-one, (huile), à partir de 7-(2,2-diméthyl-1,3-dioxol-4-yl méthoxy)-5-hydroxy-2-(3-hydroxy-4-méthoxy-2-propyl phényl)-4H-1-benzopyran-4-one, (cf la demande de brevet EP 709 383) et de Br-CH₂-COOC₂H₅ et NaH.
g) 6-allyl-7-(2,2-diméthyl-1,3- dioxol-4-yl méthoxy)-5-éthoxycarbonylméthoxy-2-(2-allyl-3-éthoxycarbonylméthoxy-4-méthoxy phényl)-4H-1-benzopyran-4-one, (huile), à partir de 6-allyl-7-(2,2-diméthyl-1,3-dioxol-4-yl méthoxy)-5-hydroxy-2-(2-allyl-3-hydroxy-4-méthoxy phényl)-4H-1-benzopyran-4-one (cf la demande de brevet EP 709 383) et de BrCH₂COOC₂H₅ et NaH.
h) 6-allyl-7-(2,2-diméthyl-1,3-dioxol-4-yl méthoxy)-5-hydroxy-2-(2-allyl-3-éthoxycarbonylméthoxy-4-méthoxy phényl)-4H-1-benzopyran-4-one, (huile), à partir de 6-allyl-7-(2,2-diméthyl-1,3-dioxol-4-yl méthoxy)-5-hydroxy-2-(2-allyl-3-hydroxy-4-méthoxy phényl)-4H-1-benzopyran-4-one, (cf la demande de brevet EP 709 383) et de Br-CH₂-COOC₂H₅.
i) 7-allyloxy-5-hydroxy-2-(3-éthoxycarbonylméthoxy-4-méthoxy phényl)-4H-1-benzopyran-4-one, P.F. : 151°C, à partir de 7-allyloxy-5-hydroxy-2-(3-hydroxy-4-méthoxy phényl)-4H-1-benzopyran-4-one (cf la demande de brevet EP 709 383) et de Br-CH₂-COOC₂H₅.
j) 5,7-di-éthoxycarbonylméthoxy-2-(3-éthoxycarbonylméthoxy-4-méthoxy phényl)-4H-1-benzopyran-4-one, P.F. : 150°C, à partir de diométine et de Br-CH₂-COOC₂H₅ et NaH.
k) 7-allyloxy-5-éthoxycarbonylméthoxy-2-(3-éthoxycarbonylméthoxy-4-méthoxy phényl)-4H-1-benzopyran-4-one, P.F. : 149-150°C, à partir de 7-allyloxy-5-hydroxy-2-(3-éthoxycarbonylméthoxy-4-méthoxy phényl)-4H-1-benzopyran-4-one (cf la demande de brevet EP 709 383) et de Br-CH₂-COOC₂H₅ et NaH.
l) 6,8-diallyl-7-éthoxycarbonylméthoxy-5-hydroxy-2-(2-allyl-3-éthoxycarbonyl méthoxy-4-méthoxy phényl)-4H-1-benzopyran-4-one PF : 98 °C, à partir de 6,8 - diallyl - 5,7 - dihydroxy - 2- (3-hydroxy-4-méthoxy phényl) - 4H - 1 - benzopyran-4-one (cf la demande de brevet EP 709 383) et de Br-CH₂-COOC₂H₅.

### β) Deuxième méthode

Le composé titre du stade A a également été préparé de la manière suivante : 340 g de 5,7-dihydroxy-2-(3-allyloxy-4-méthoxy phényl)-4H-1-benzopyran-4-one (cf la demande de brevet EP 94.12783) sont traités par 165 g de K₂CO₃ dans 5100 ml de diméthylformamide à 50°C pendant 10 heures. On ajoute ensuite 200 g de bromoacétate d'éthyle et porte à 80°C pendant 15 heures. On laisse revenir à température ambiante et filtre les minéraux. Le filtrat est concentré à sec par distillation des éléments volatils sous pression réduite. Le résidu est filtré sur silice en éluant au dichlorométhane. Le produit obtenu est recristallisé dans l'isopropanol pour donner, avec un rendement de 79%, 338 g du composé titre du stade A à l'état pur, P.F. : 171°C.

De la même manière ont été préparés les composés suivants :
m) 5,7-di-éthoxycarbonylméthoxy-2-(3-allyloxy-4-méthoxy phényl)-4H-1-benzopyran-4-one, P.F. : 120°C, à partir de 5,7-dihydroxy-2-(3-allyloxy-4-méthoxy phényl)-4H-1-benzopyran-4-one, (cf la demande de brevet EP 709 383), de BrCH₂COOC₂H₅ et de NaH.
n) 5,7-diallyloxy-2-(4-méthoxy-3-éthoxycarbonylméthoxy phényl)-4H-1-benzopyran-4-one, P.F. : 123 °C, à partir de 7-allyloxy-5-hydroxy-2-(4-méthoxy-3-éthoxy-carbonylméthoxy phényl)-4H-1-benzopyran-4-one (décrit dans la présente demande), de bromure d'allyle et de NaH.
o) 5-éthoxycarbonylméthoxy-7-allyloxy-2-(3-allyloxy-4-méthoxy phényl)-4H-1-benzopyran-4-one, PF : 107 °C, à partir de 7-allyloxy-5-hydroxy-2-(3-allyloxy-4-méthoxy- phényl)-4H-1-benzopyran-4-one (cf la demande de brevet EP 709 383), de Br-CH₂-COOC₂H₅ et de NaH.

### Stade B : 7-éthoxycarbonylméthoxy-5-hydroxy-2-(2-allyl-3-hydroxy-4-méthoxy phényl)-4H-1-benzopyran-4-one

42,6 g de 7-éthoxycarbonylméthoxy-5-hydroxy-2-(3-allyloxy-4-méthoxyphényl)-4H-1-benzopyran-4-one, (produit titre du stade A) sont portés à reflux dans 430 ml de 1,2,4-trichlorobenzène pendant 1 heure 30, sous atmosphère d'azote. le milieu réactionnel est ensuite ramené à température ambiante et dilué avec 1000 ml d'éther de pétrole. Après une heure d'agitation le précipité est recueilli par filtration puis recristallisé dans l'isopropanol. On obtient ainsi 34 g du composé titre du stade B à l'état pur, P.F. : 155°C. De la même manière, ont été préparés les composés suivants :
a) 5,7-di-éthoxycarbonylméthoxy-2-(3-hydroxy-4-méthoxy-2-allyl phényl)-4H-1-benzopyran-4-one, P.F. : 125-127°C, à partir de 5,7-di-éthoxycarbonylméthoxy-2-(3-allyloxy-4-méthoxyphényl)-4H-1-benzopyran-4-one (décrit dans la présente demande).
b) 5-(2,2-diméthyl-1,3-dioxol-4-yl méthoxy)-7-éthoxycarbonylméthoxy-2-(2-allyl-3-hydroxy-4-méthoxy phényl)-4H-1-benzopyran-4-one, P.F. : 85°C, à partir de 5-(2,2-diméthyl-1,3-dioxol-4-yl méthoxy)-7-éthoxycarbonylméthoxy-2-(3-allyloxy-4-méthoxy phényl)-4H-1-benzopyran-4-one (décrit dans la présente demande).
c) 5,7-dihydroxy-8-allyl-2-(3-éthoxycarbonylméthoxy-4-méthoxy phényl)4H-1-benzopyran-4-one, PF : 224 °C.
d) 5,7-dihydroxy-6-allyl-2-(3-éthoxycarbonylméthoxy-4-méthoxy phényl)4H-1-benzopyran-4-one, PF : 239 °C.
   Les deux derniers composés objet de c) et d) ont été chacun obtenus sous forme de mélange et séparés chromatographiquement sur colonne de silice avec CH₂Cl₂/C₂H₅OH 98/2 comme éluant, à partir de 7-allyloxy-5-hydroxy-2-(3-éthoxycarbonylméthoxy-4-méthoxy phényl)-4H-1-benzopyran -4-one (décrit dans la présente demande).
e) 5,7-dihydroxy-6,8-diallyl-2-(3-éthoxycarbonylméthoxy-4-méthoxy phényl)-4H-1-benzopyran-4-one, PF : 190 °C, à partir de 5,7-diallyloxy-2-(3-éthoxycarbonylméthoxy-4-méthoxy phényl)-4H-1-benzopyran-4-one.

La réaction de transposition peut également être effectuée en position para, par exemple pour préparer le composé suivant :
f) 7-éthoxycarbonylméthoxy-5-hydroxy-2-[5-hydroxy-4-méthoxy-2-(3-méthylbut-2-ényl)phényl]-4H-1-benzopyran-4-one. 13 g de 7-éthoxycarbonylméthoxy-5-hydroxy-2-[4-méthoxy-3-(3-méthylbut-2-ényloxy)phényl]-4H-1-benzopyran-4-one, sont portés à reflux dans 130 ml de N,N-diméthylaniline pendant 2 heures. La plus grosse partie du solvant (90%) est éliminée par distillation sous pression réduite. L'huile résiduelle est traitée à température ambiante par HCI dilué et le mélange est extrait à l'éther éthylique. La phase éthérée, lavée à l'eau est séchée puis amenée à sec. Le résidu est chromatographié sur silice pour donner après élution au dichlorométhane, avec un rendement de 38%, 4,9 g du produit attendu sous forme d'huile pratiquement pure.

### Stade C : 7-éthoxycarbonylméthoxy-5-hydroxy-2-(3-hydroxy-4-méthoxy-2-propyl phényl)-4H-1-benzopyran-4-one

21,3 g de 7- éthoxycarbonylméthoxy-5-hydroxy-2-(2-allyl-3-hydroxy-4-méthoxy phényl)-4H-1-benzopyran-4-one sont dissous dans 300 ml de diméthylformamide. La solution est versée sur 2 g de charbon palladié à 10% de palladium. Le tout est hydrogéné à 50°C sous une pression de 63.10⁴ Pa jusqu'à absorption de la quantité théorique d'hydrogène.
Le mélange est ensuite filtré sur millipore et le solvant est éliminé par distillation sous pression réduite. Le résidu brut (21 g) peut être utilisé tel quel. Un échantillon analytique, obtenu par recristallisation dans l'isopropanol fond à 138°C.

De la même manière ont été obtenus les composés suivants :
a) 7-éthoxycarbonylméthoxy-5-hydroxy-2-(4-méthoxy-3-propyloxy phényl) -4H-1-benzopyran-4-one, P.F. : 157°C, à partir de 7-éthoxycarbonylméthoxy-5-hydroxy-2-(3-allyloxy-4-méthoxy phényl)-4H-1-benzopyran-4-one (décrit dans la présente demande).
b) 5,7-di-éthoxycarbonylméthoxy-2-(3-hydroxy-4-méthoxy-2-propyl phényl) -4H-1-benzopyran-4-one, P.F. : 72°C, à partir de 5,7-di-éthoxycarbonylméthoxy-2-(2-allyl-3-hydroxy-4-méthoxy phényl)-4H-1-benzopyran-4-one (décrit dans la présente demande).
c) 7-éthoxycarbonylméthoxy-5-hydroxy-2-[3-hydroxy-4-méthoxy-6-(3-méthylbutyl)phényl]-4H-1-benzopyran-4-one, huile, à partir de 7-éthoxycarbonyl méthoxy-5-hydroxy-2-[3-hydroxy-4-méthoxy-6-(3-méthylbut-2-ényl)phényl] -4H-1-benzopyran-4-one (décrit dans la présente demande).
d)5,7-dihydroxy-6-propyl-2-(3-éthoxycarbonylméthoxy-4-méthoxy phényl)4H-1-benzopyran-4-one, PF : 216 °C, à partir de 5,7-hydroxy-6-allyl-2-(3-éthoxycarbonylméthoxy - 4 - méthoxy phényl) - 4H-1-benzopyran-4-one (décrit dans la présente demande).
e) 5,7-dihydroxy-8-propyl-2-(3-éthoxycarbonylméthoxy-4-méthoxy phényl)4H-1-benzopyran-4-one, PF : 190 °C, à partir de 5,7-dihydroxy-8-allyl-2-(3-éthoxycarbonylméthoxy-4-méthoxy phényl)-4H-1-benzopyran-4-one (décrit dans la présente demande).
f) 5,7-dihydroxy-6,8-dipropyl-2-(3-éthoxycarbonylméthoxy-4-méthoxy phényl)-4H-1-benzopyran-4-one, PF : 205 °C, à partir de 5,7-dihydroxy-6,8-diallyl-2-(3-éthoxycarbonylméthoxy-4-méthoxy phényl)-4H-1-benzopyran-4-one (décrit dans la présente demande).

### Stade D : Produit titre de l'exemple 1

10,7 g de 7-éthoxycarbonylméthoxy-5-hydroxy-2-(3-hydroxy-4-méthoxy-2-propyl phényl)-4H-1-benzopyran-4-one sont portés au reflux pendant 1 heure dans un mélange de 44 ml de soude N et de 88 ml d'éthanol. L'éthanol est ensuite éliminé par distillation et le mélange aqueux est filtré à chaud sur filtre millipore. Le filtrat basique est alors acidifié à pH = 1 avec une solution d'HCIN. Le précipité formé est recueilli par filtration, séché puis recristallisé dans un mélange eau/acétone. On obtient ainsi 9 g du composé titre de l'exemple 1 à l'état pur, P.F. : 251°C.
De la même manière, ont été préparés les acides suivants, à partir des esters éthyliques correspondants tous inclus dans la présente demande :
a) 7-carboxyméthoxy-5-hydroxy-2-(3-allyloxy-4-méthoxy phényl)-4H-1-benzopyran-4-one, P.F. : 253-255°C.
b) 7-carboxyméthoxy-5-hydroxy-2-(4-méthoxy-3-propyloxy phényl)-4H-1-benzopyran-4-one, P.F. : 259°C.
c) 7-carboxyméthoxy-5-hydroxy-2-(2-allyl-3-hydroxy-4-méthoxy phényl)-4H-1-benzopyran-4-one, P.F. : 264°C.
d) 5,7-dicarboxyméthoxy-2-(2-allyl-3-hydroxy-4-méthoxy phényl)-4H-1-benzopyran-4-one, P.F. : 247°C.
e) 5,7-dicarboxyméthoxy-2-(3-hydroxy-4-méthoxy-2-propyl phényl)-4H-1-benzopyran-4-one, P.F. : 255°C.
f) 7-carboxyméthoxy-5-hydroxy-2-[3-hydroxy-4-méthoxy-6-(3-méthybut-2-ényl)phényl]-4H-1-benzopyran-4-one, P.F. : 210°C.
g) 5,7-dicarboxyméthoxy-2-(3-carboxyméthoxy-4-méthoxy phényl)-4H-1-benzopyran-4-one, P.F. : 300°C.
h) 6,8-di-allyl-7-carboxyméthoxy-5-hydroxy-2-(2-allyl-3-hydroxy-4-méthoxy phényl)-4H-1-benzopyran-4-one, P.F. : 209°C.
i) 5,7-dihydroxy-6,8-dipropyl-2-(3-carboxyméthoxy-4-méthoxy phényl)-4H-1-benzopyran-4-one, PF : 232 °C.
j) 7-carboxyméthoxy-6,8-diallyl-5-hydroxy-2-(2-allyl-3-carboxyméthoxy-4-méthoxy phényl)-4H-1-benzopyran-4-one, PF : 91 °C.
k) 5,7-dihydroxy-6-propyl-2-(3-carboxyméthoxy-4-méthoxy phényl)-4H-1-benozpyran-4-one, PF : 259 °C.
l) 5,7-dihydroxy-8-propyl-2-(3-carbonyméthoxy-4-méthoxyphényl-4H-1-benzopyran-4-one, PF : 250 °C.
m) 8-allyl-5,7-dihydroxy-2-(3-carboxyméthoxy-4-méthoxyphényl)-4H-1-benzopyran-4-one, PF : 223 °C.
n) 6-allyl-5,7-dihydroxy-2-(3-carboxyméthoxy-4-méthoxy phényl)-4H-1 benzopyran-4-one, PF : 265 °C.
o) 6,8-diallyl-5,7-dihydroxy-2-(3-carboxyméthoxy-4-méthoxy phényl)-4H-1-benzopyran-4-one, PF : 200 °C.
p) 7-carboxyméthoxy-5-hydroxy- 2 - [ 2 - (3-méthyl n-butyl) - 5 - hydroxy - 4-méthoxy phényl]-4H-1-benzopyran-4-one, PF : 159 °C.

Ces acides peuvent être transformés en sel de sodium comme par exemple :
q) le sel de sodium de la 7-carboxyméthoxy-5-hydroxy-2-(2-allyl-3-hydroxy-5-méthoxy phényl)-4H-1-benzopyran-4-one : 3,98 g de 7-carboxyméthoxy-5-hydroxy-2-(2-allyl-3-hydroxy-4-méthoxy phényl)-4H-1-benzopyran-4-one sont dissous dans 10 ml de soude N et 390 ml d'eau. La solution est rendue limpide par filtration sur filtre millipore. Le filtrat est amené à sec par distillation de l'eau sous pression réduite et le résidu est recristallisé dans un mélange eau/éthanol. On obtient ainsi 3,5 g du sel de sodium attendu, P.F. > 260°C.

L'hydrolyse peut aussi concerner simultanément un ester et un acétonide, comme par exemple pour préparer le composé suivant :
r) 7-(2,3-dihydroxypropyloxy)-5-hydroxy-2-(3-carboxyméthoxy-4-méthoxy-2-propyl phényl)-4H-1-benzopyran-4-one :
   5,42 g de 7-(2,2-diméthyl-1,3-dioxol-4-yl)méthoxy-5-hydroxy-2-(3-éthoxycarbonylméthoxy-4-méthoxy-2-propyl phényl)-4H-1-benzopyran-4-one sont portés à reflux dans un mélange de 12 ml de soude N et 80 ml d'éthanol pendant 1 heure. On ajoute ensuite au mélange 10 ml d'eau et 32 ml d'acide acétique et chauffe à reflux jusqù'à dissolution complète (environ 15 à 30 minutes), puis on laisse revenir à température ambiante. Le précipité formé est filtré, lavé à l'eau puis recristallisé dans l'acétone. On obtient ainsi 3 g du composé attendu à l'état pur, P.F. : 135-145°C.

De la même manière ont été préparés les composés suivants :
s) 7-(2,3-dihydroxypropyloxy)-5-hydroxy-2-(3-carboxyméthoxy-4-méthoxy phényl)-4H-1-benzopyran-4-one, P.F. : 217°C, à partir de 7-(2,2-diméthyl-1,3-dioxol-4-yl méthoxy-5-hydroxy-2-(3-éthoxycarbonylméthoxy-4-méthoxy phényl)-4H-1-benzopyran-4-one (décrit dans la présente demande).
t) 5-carboxyméthoxy-7-(2,3-dihydroxy propyloxy-2-(3-carboxyméthoxy-4-méthoxy phényl)-4H-1-benzopyran-4-one, P.F. : du dihydrate correspondant : 161°C, à partir de 7-(2,2-diméthyl-1,3-dioxol-4-yl méthoxy)-5-éthoxycarbonylméthoxy-2-(3-éthoxycarbonylméthoxy-4-méthoxy phényl)-4H-1-benzopyran-4-one (décrit dans la présente demande).
u) 5-carboxyméthoxy-7-(2,3-dihydroxy propyloxy)-2-(3-carboxyméthoxy-4-méthoxy-2-propyl phényl)-4H-1-benzopyran-4-one, P.F. > 250°C, à partir de 7-(2,3-diméthyl-1,3-dioxol-4-yl-méthoxy)-5-éthoxycarbonylméthoxy-2-(3-éthoxycarbonylméthoxy-4-méthoxy-2-propyl phényl)-4H-1-benzopyran-4-one (décrit dans la présente demande).
v) 6-allyl-7-(2,3-dihydroxy propyloxy)-5-hydroxy-2-(2-allyl-3-carboxyméthoxy-4-méthoxy phényl)-4H-1-benzopyran-4-one, P.F. : 139-140°C, à partir de 6-allyl-7-(2,2-diméthyl-1,3-dioxol-4-yl-méthoxy)-5-hydroxy-2-(2-allyl-3-éthoxycarbonyl méthoxy-4-méthoxy phényl)-4H-1-benzopyran-4-one (décrit dans la présente demande).
w) 7-carboxyméthoxy-5-(2,3-dihydroxypropyloxy)-2-(2-allyl-3-hydroxy-4-méthoxy phényl)-4H-1-benzopyran-4-one, P.F. : 236°C, à partir de 5-(2,2-diméthyl-1,3-dioxol-4-yl méthoxy)-7-éthoxycarbonylméthoxy-2-(2-allyl-3-hydroxy-4-méthoxy phényl)-4H-1-benzopyran-4-one (décrit dans la présente demande).

Ces acides peuvent également être convertis en sel de sodium, comme par exemple :
x) le sel de sodium de la 7-(2,3-dihydroxypropyloxy)-5-hydroxy-2-(3-carboxyméthoxy-4-méthoxy-2-propyl phényl)-4H-1-benzopyran-4-one : 4,74 g de 7-(2,3-dihydroxy propyloxy)-5-hydroxy-2-(3-carboxyméthoxy-4-méthoxy-2-propyl phényl)-4H-1-benzopyran-4-one sont dissous dans 10 ml de soude N et 200 ml d'eau. Le mélange est filtré sur filtre millipore. Le filtrat est amené à sec. Le résidu obtenu est recristallisé dans l'acétone. On obtient ainsi 4 g de sel de sodium attendu, P.F. : 245-246°C.

En opérant de la même manière, on peut obtenir un sel de calcium d'un diacide, comme par exemple :
y) le sel de calcium de la 5-carboxyméthoxy-7-(2,3-dihydroxypropyloxy)-2-(3-carboxyméthoxy-4-méthoxy-2-propyl phényl)-4H-1-benzopyran-4-one, qui recristallisé dans un mélange eau/acétone fond au-dessus de 250°C.

### Exemple 2 :

### Etude pharmacologique

### 1/ Activité anti-hyperperméabilité

Elle a été déterminée par l'effet des composés de l'invention sur l'extravasation de FITC-dextran dans la microcirculation du hamster cheek pouch.
Le hamster cheek pouch est un modèle expérimental permettant d'étudier de façon quantitative la perméabilité de macromolécules en utilisant du dextran marqué à la fluorescéine (FITC-dextran).
Les expériences ont été effectuées sur des hamsters mâles dont le poids est de 85 à 120 g. Les animaux sont anesthésiés avec du pentobarbical sodique (60 mg/kg i.p.). Pendant l'expérience, l'anesthésie est maintenue par de l'α-chloralose (100 mg/kg) introduite par un cathéter dans l'artère fémorale. Les animaux respirent spontanément par une canule trachéale et la température corporelle est maintenue à 37,5°C à l'aide d'un "heating pad" contrôlé par un thermistor rectal.

Après anesthésie initiale, le cheek pouch est préparé selon la méthode de Duling (Microvasc. Res., 5, 423-429, 1973) et Svensjö et al (Uppsala. J. Med. Sci.,83, 71-79, 1978). La préparation est montée dans une cuve expérimentale et superfusée à 6 ml/min par une solution physiologique contenant du Hepes. La température de la solution physiologique est maintenue à 36,5°C et un courant de gaz (5% CO₂ -95% N₂) est appliqué au-dessus de la cuve expérimentale afin de maintenir le niveau de la pO₂ de la solution entre 12 et 15 mmHg et le pH à 7,4.

Trente minutes après le montage de la préparation, le FITC-dextran (150 000 dalton, 50 mg/ml) est administré par voie intraveineuse chez l'animal à la dose de 250 mg/kg. La préparation est observée par la méthode de "intravital microscopy" en lumière UV à l'aide d'un microscope Ortholux II de Leitz.

Après l'injection de FITC-dextran, la préparation est soumise à une application locale d'un agent induisant la perméabilité, l'histamine à la concentration de 2 x 10⁻⁶ M.

L'application de ces agents et de la période d'ischémie provoque des "fuites" (visibles comme des petites plaques fluorescentes) dans toute la préparation. Le nombre de fuites est déterminé à plusieurs intervalles après l'administration de l'histamine (de 2 à 30 mn) et exprimé en nombre de fuites par cm². Les préparations sont exposés à plusieurs reprises (séparées d'au moins 30 minutes) à une application locale d'histamine.

Deux types d'études ont été effectués afin de déterminer l'activité per os des composés de la présente invention.

Les animaux sont traités par gavage, trente minutes avant l'anesthésie, à la dose de 100 mg/kg d'un des composés de l'invention mis en suspension dans la gomme arabique. La dose du produit est contenue dans 0,2 ml. Les animaux contrôles reçoivent un gavage avec 0,2 ml de gomme arabique (placébo).
Les composés de l'invention administrés à 100 mg/kg p.o. chez le hamster, diminuent de façon significative (t-test ; *p<0,05) et durable, l'extravasation de macromolécules provoquée par l'histamine dans la microcirculation. Les résultats obtenus avec l'histamine, 3 heures après l'administration des produits, sont récapitulés dans le tableau 1. Ces résultats sont supérieurs à ceux obtenus dans les mêmes conditions opératoires, avec la substance de référence, la Troxérutine.

**Tableau 1**

| *Influence des composés de l'invention sur l'extravasation de FITC-dextran dans le hamster cheek pouch après traitement per os (100 mg/kg)* | | |
|---|---|---|
| ***Produits*** | ***Nombre de fuites par cm***^{**2**} | |
| | Non traités | 3 h après traitement |
| **Composé de l'exemple 1** | 343 | 251 |
| **Composé de l'exemple 1 Stade D, b)** | 394 | 295 |
| **Composé de l'exemple 1 Stade D, c)** | 343 | 227 |
| **Composé de l'exemple 1 Stade D, d)** | 374 | 313 |
| **Composé de l'exemple 1 Stade D, e)** | 374 | 287 |
| **Composé de l'exemple 1 Stade D, g)** | 374 | 294 |
| **Composé de l'exemple 1 Stade D, m)** | 368 | 324 |
| **Composé de l'exemple 1 Stade D, o)** | 368 | 191 |
| **Composé de l'exemple 1 Stade D, p)** | 443 | 363 |
| **Composé de l'exemple 1 Stade D, s)** | 443 | 354 |
| **Composé de l'exemple 1 Stade D, u)** | 374 | 239 |
| **Composé de l'exemple 1 Stade D, v)** | 374 | 190 |

## Revendications

1. Les acides et esters de la diosmétine de formule (I) : 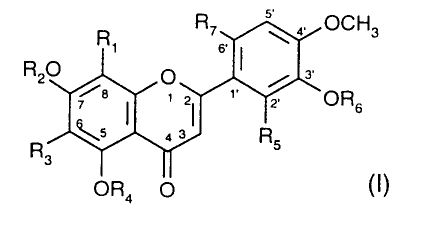 dans laquelle :
- R₂, R₄ et R₆, identiques ou différents, représentent chacun un atome d'hydrogène, un radical alkyle de 1 à 5 atomes de carbone en chaine droite ou ramifiée, ou un radical de formule : 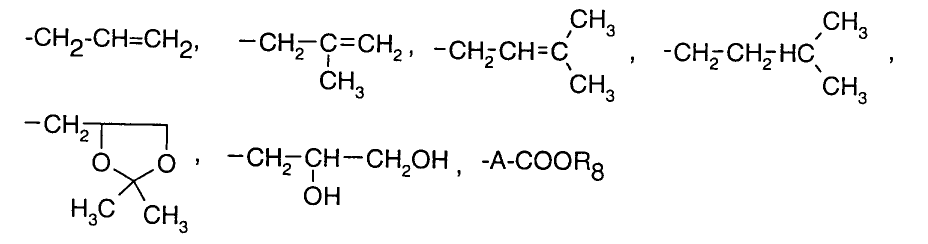 dans lesquelles :
· A représente une chaîne hydrocarbonée droite de contenant de 1 à 3 atomes de carbone éventuellement substituée par un radical méthyle, ou par un radical hydroxy ;
· R₈ représente un atome d'hydrogène ou un radical alkyle de 1 à 5 atomes de carbone et
- R₃ et R₅, identiques ou différents représentent chacun un atome d'hydrogène ou un radical de formule : 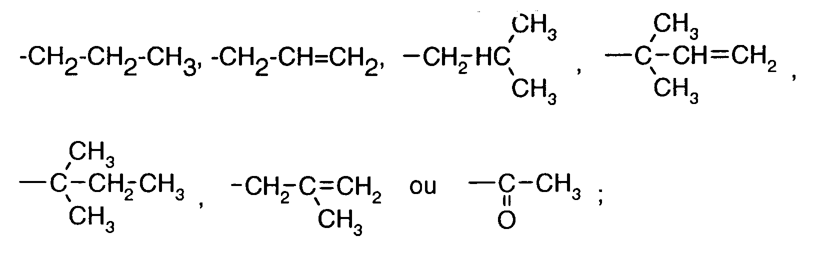 et
- R₁ et R₇, identiques ou différents, représentent chacun un atome d'hydrogène ou un radical de formule : 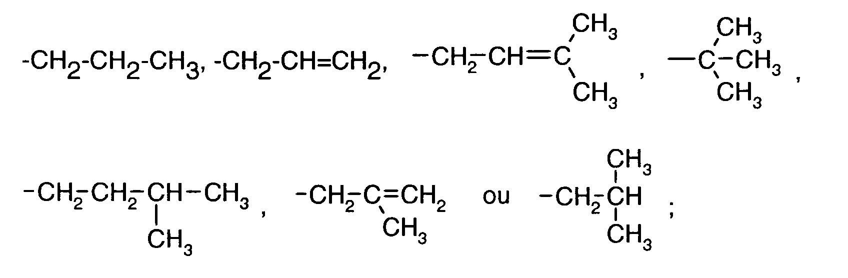
- à condition toutefois que :
- l'un au moins des substituants R₂, R₄ et R₆ représente le groupe -A-COOR₈, et
- lorsque R₂, et/ou R₄, et/ou R₆ représente -A-COOR₈, (A et R₈ ayant les significations précédemment définies), alors un au moins des substituants restants, parmi R₁ à R₇, ne représente pas un atome d'hydrogène;
ainsi que leurs énantiomères quand ils existent et leurs sels physiologiquement tolérables avec des bases appropriées.

2. Un composé de la revendication 1 qui est la 7-carboxyméthoxy-5-hydroxy-2-(3-hydroxy-4-méthoxy-2-propylphényl)-4H-1-benzopyran-4-one.

3. Un composé de la revendication 1 qui est la 7-carboxyméthoxy-5-hydroxy-2-(4-méthoxy-3-propyloxy phényl)-4H-1-benzopyran-4-one.

4. Un composé de la revendication 1 qui est la 7-carboxyméthoxy-5-hydroxy-2-(2-allyl-3-hydroxy-4-méthoxy phényl)-4H-1-benzopyran-4-one.

5. Les compositions pharmaceutiques contenant comme principe actif un composé selon l'une quelconque des revendications 1 à 4, mélangé ou associé à un ou plusieurs excipients pharmaceutiquement appropriés.

6. Les compositions pharmaceutiques selon la revendication 5 présentées sous une forme convenant dans le traitement de l'insuffisance veineuse chronique et dans toute autre maladie entraînant une activité d'hyperperméabilité et/ou d'inflammation.

## Claims

1. Diosmetin acids and esters of formula (I) : 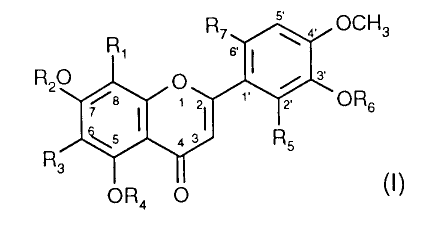 wherein:
- R₂, R₄ and R₆, which are identical or different, each represents a hydrogen atom, an alkyl radical having from 1 to 5 carbon atoms in straight or branched chain, or a radical of formula : 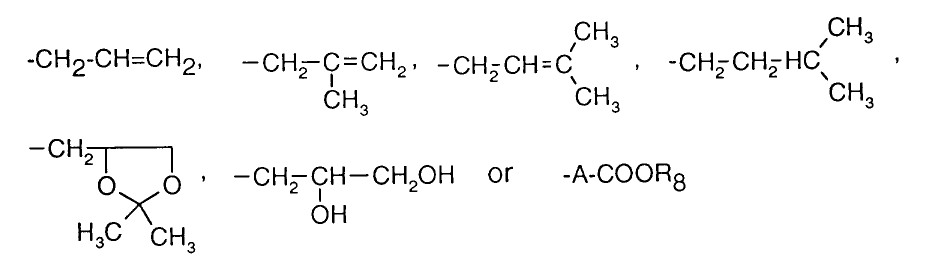 wherein :
· A represents a straight hydrocarbon chain having from 1 to 3 carbon atoms and optionally substituted by a methyl radical, or by a hydroxy radical;
· R₈ represents a hydrogen atom or an alkyl radical having from 1 to 5 carbon atoms and
- R₃ and R₅, which are identical or different, each represents a hydrogen atom or a radical of formula : 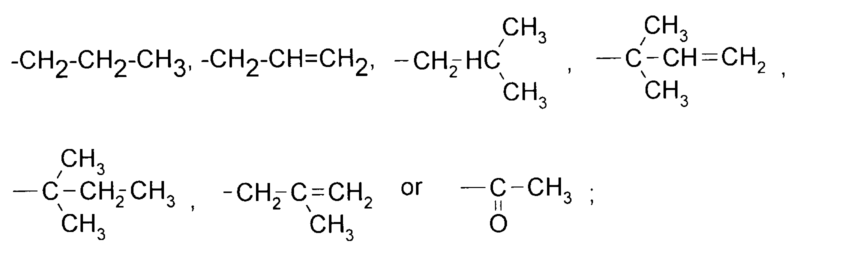 and
- R₁ and R₇, which are identical or different, each represents a hydrogen atom or a radical of formula : 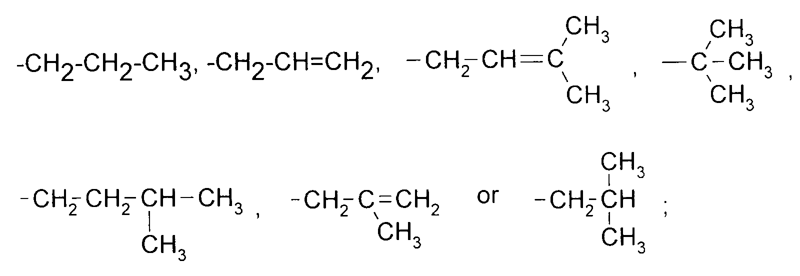
- with the proviso that :
· at least one of the substituents R₂, R₄ and R₆ represents the group -A-COOR₈, and
- when R₂ and/or R₄ and/or R₆ represent(s) -A-COOR₈ (A and R₈ being as defined above), then at least one of the remaining substituents among R₁ to R₇ does not represent a hydrogen atom;
and also the enantiomers thereof, where they exist, and the physiologically tolerable salts thereof with appropriate bases.

2. A compound of claim 1 which is 7-carboxymethoxy-5-hydroxy-2-(3-hydroxy-4-methoxy-2-propylphenyl)-4H-1-benzopyran-4-one.

3. A compound of claim 1 which is 7-carboxymethoxy-5-hydroxy-2-(4-methoxy-3-propoxyphenyl)-4H-1-benzopyran-4-one.

4. A compound of claim 1 which is 7-carboxymethoxy-5-hydroxy-2-(2-allyl-3-hydroxy-4-methoxyphenyl)-4H-1-benzopyran-4-one.

5. Pharmaceutical compositions comprising as active ingredient a compound according to any one of claims 1 to 4, in admixture or association with one or more pharmaceutically appropriate excipients.

6. Pharmaceutical compositions according to claim 5, presented in a form suitable for use in the treatment of chronic venous insufficiency and any other disorder involving hyperpermeability and/or inflammation.

## Patentansprüche

1. Diosmetinsäuren und -ester der Formel (I): 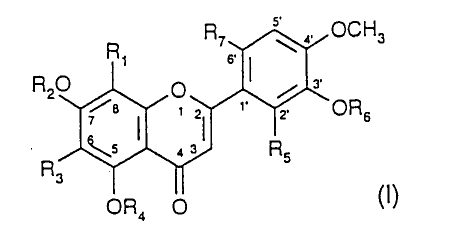 in der:
- R₂, R₄ und R₆, die gleichartig oder verschieden sind, jeweils ein Wasserstoffatom, eine Alkylgruppe mit 1 bis 5 Kohlenstoffatomen in gerader oder verzweigter Kette, oder eine Gruppe der Formel: 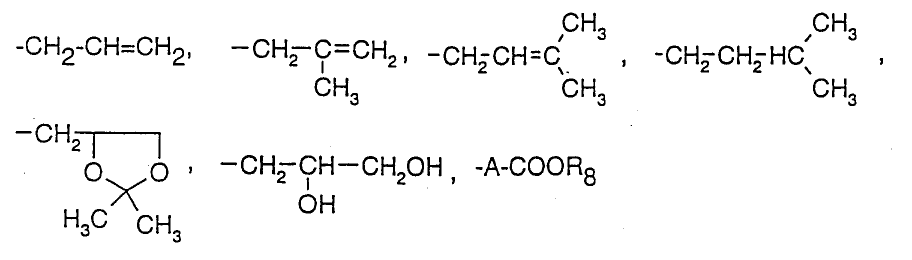 worin:
· A eine geradkettige Kohlenwasserstoffkette mit 1 bis 3 Kohlenstoffatomen, die gegebenenfalls durch eine Methylgruppe oder durch eine Hydroxygruppe substituiert ist; und
· R₈ ein Wasserstoffatom oder eine Alkylgruppe mit 1 bis 5 Kohlenstoffatomen darstellen,
- R₃ und R₅, die gleichartig oder verschieden sind, jeweils ein Wasserstoffatom oder eine Gruppe der Formel: 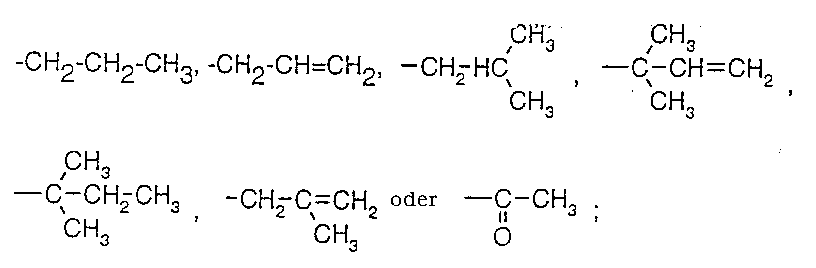 und
- R₁ und R₇, die gleichartig oder verschieden sind, jeweils ein Wasserstoffatom oder eine Gruppe der Formel: 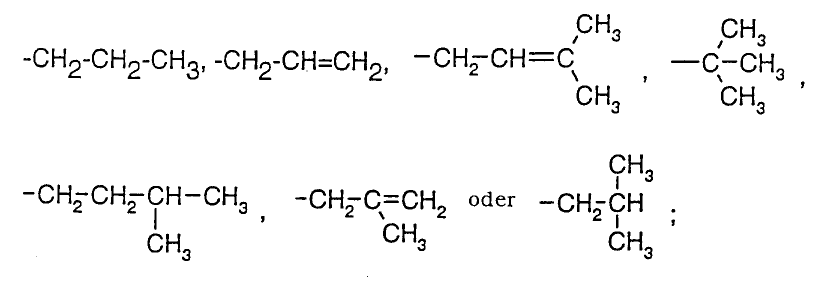
- mit der Maßgabe bedeuten, daß:
- mindestens einer der Substituenten R₂, R₄ und R₆ eine Gruppe -A-COOR₈ darstellt und
- wenn R₂ und/oder R₄ und/oder R₆ -A-COOR₈ bedeutet (worin A und R₈ die oben angegebenen Bedeutungen besitzen) mindestens einer der verbleibenden Substituenten der Gruppe R₁ bis R₇ kein Wasserstoffatom darstellt;
sowie deren eventuell existierende Enantiomere und deren physiologisch verträgliche Salze mit geeigneten Säuren.

2. Verbindung nach Anspruch 1, nämlich 7-Carboxymethoxy-5-hydroxy-2-(3-hydroxy-4-methoxy-2-propylphenyl)-4H-1-benzopyran-4-on.

3. Verbindung nach Anspruch 1, nämlich 7-Carboxymethoxy-5-hydroxy-2-(4-methoxy-3-propyloxy-phenyl)-4H-1-benzopyran-4-on.

4. Verbindung nach Anspruch 1, nämlich 7-Carboxymethoxy-5-hydroxy-2-(2-allyl-3-hydroxy-4-methoxy-phenyl)-4H-1-benzopyran-4-on.

5. Pharmazeutische Zubereitungen enthaltend als Wirkstoff eine Verbindung nach einem der Ansprüche 1 bis 4 in Mischung oder in Kombination mit einem oder mehreren pharmazeutisch geeigneten Trägermaterialien.

6. Pharmazeutische Zubereitungen nach Anspruch 5 in einer Form, die zur Behandlung von chronischer Veneninsuffizienz und sämtlichen anderen Erkrankungen, die eine Hyperpermeabilitäts- und / oder Entzündungsaktivität involvieren, geeignet ist.
